# EUROPÄISCHE PATENTSCHRIFT

(11) **EP 2 651 861 B1**
(45) Veröffentlichungstag und Bekanntmachung des Hinweises auf die Patenterteilung: **25.02.2015**
(21) Anmeldenummer: 11802354.8
(22) Anmeldetag: 15.12.2011
(51) Int. Cl.: C07C 13/04, C07C 215/08

(54) **VERFAHREN ZUR HERSTELLUNG EINES N,N-DIALKYL-ETHANOLAMINS MIT HOHER FARBSTABILITÄT**
PROCESS FOR PREPARING AN N,N-DIALKYLETHANOLAMINE HAVING HIGH COLOUR STABILITY
PROCÉDÉ DE PRÉPARATION D'UNE N,N-DIALKYL-ÉTHANOLAMINE PRÉSENTANT UNE GRANDE STABILITÉ DE LA COULEUR

(30) Priorität: 17.12.2010 EP 10195662
(43) Veröffentlichungstag der Anmeldung: 23.10.2013
(73) Patentinhaber: BASF SE, 67056 Ludwigshafen (DE)
(72) Erfinder: PAPE, Frank-Friedrich, 67259 Kleinniedesheim (DE); MELDER, Johann-Peter, 67459 Böhl-Iggelheim (DE); KRAUSE, Alfred, 67346 Speyer (DE); BOU CHEDID, Roland, 68159 Mannheim (DE); RUDLOFF, Martin, 67161 Gönnheim (DE)
(86) Internationale Anmeldenummer: PCT/EP2011/072936
(87) Internationale Veröffentlichungsnummer: WO 2012/080409

(56) Entgegenhaltungen:
- US-A1- 2004 068 143

## Beschreibung

Die vorliegende Erfindung betrifft ein Verfahren zur Herstellung eines N,N-Dialkyl-ethanolamins mit hoher Farbstabilität.

N,N-Dialkyl-ethanolamine sind wichtige Zwischenprodukte für die chemische und pharmazeutische Industrie. Dimethylethanolamin findet z. B. in Form von Salzen, Seifen, Ether und Ester als Emulgator und oberflächenaktive Substanz sowie als Katalysator in der Polyurethanchemie Anwendung auf den verschiedensten Gebieten. In der pharmazeutischen Industrie wird es zur Synthese von Wirkstoffen (Tranquilizer, Antihistaminika und Analgetika) benutzt. Verfärbungen in N,N-Dialkyl-ethanolaminen, wie Dimethylethanolamin, sind bei den meisten Anwendungen unerwünscht.

Die Anlagerung fast aller Amine an Ethylenoxid (EO) wird, wie die des Ammoniaks, durch Zusatz von Wasser erheblich beschleunigt. So ist zur Umsetzung von EO mit Dimethylamin (DMA) mehrstündiges Erhitzen auf 150 °C notwenig, dagegen erfolgt die Reaktion bereits in der Kälte, wenn eine wässrige DMA-Lösung verwendet wird. Eine dem Wasser ähnliche Wirkung haben auch Alkohole wie Methanol oder Ethanol. (Houben Weyl, Methoden der organischen Chemie, Band 11/1, 1957, Seite 311 ff.).

Es ist bekannt, dass Alkanolamine durch Ethoxylierung der Hydroxylgruppe zu höherethoxylierten Produkten weiterreagieren. Diese Folgereaktion kann durch Einsatz von überschüssigem Amin (1,1 : 1 bis 4 : 1) weitgehend zurückgedrängt werden (DE 23 57 076 A, DD 203 534 A (VEB Synthesewerk Schwarzheide), US 2,337,004 A, US 2,373,199 A).

Es ist weiterhin bekannt, dass tertiäre Amine, wie z. B. Dimethylethanolamin (DMEOA), sowohl in Abwesenheit als auch in Gegenwart von Wasser unterhalb von 80 °C mit Oxiranen zu thermisch labilen quartären Ammoniumverbindungen reagieren, die sich oberhalb von 90 °C mehr oder weniger rasch zersetzen. (E. Tobler et al., Helv. Chim Acta 52, 1969, Seite 408 - 418). Die Bildung dieser Basen ist in mehrfacher Hinsicht von Nachteil:
1) EO-Fixierung in den quartären Basen führt zu Selektivitätseinbußen.
2) Aufgrund ihrer hohen Basizität katalysieren die quartären Basen die Bildung höherethoxylierter Verbindungen. Dodecylamin addiert z. B. oberhalb 200 °C (--> keine quartären Basen existent) 2 Mol EO und unterhalb von 90 °C (→ quartäre Basen existent) 10 mol EO zu höherethoxylierten Verbindungen (H.L. Sanders et al., J. Am. Oil Chemists' Soc. 46, Seite 167 - 170). Insgesamt sind Selektivitätseinbußen die Folge.
3) Es wird angenommen, dass die unerwünschte Bildung von Verfärbungen im DMEOA auf komplizierte Zersetzungsmechanismen (E. Tobler et al., Helv. Chim. Acta 52, 1969, Seite 408 - 418) dieser quartären Ammoniumverbindungen zu flüchtigen ungesättigten Verbindungen, welche dann polymerisieren, zurückzuführen ist (DD 203 534 A).

Folgendes ist Stand der Technik bez. der Herstellung von farbstabilen Dialkylethanolaminen. Wasserkatalysierte Verfahren mit spezieller Aufarbeitung:

EP 70 978 A (Pennwalt Corp.): Kontinuierliche Umsetzung von überschüssigem DMA (2,2 eq) mit EO in Gegenwart von Wasser (0,2-0,5 eq) bei 150 °C; destillative Aufarbeitung unter Zusatz definierter Mengen Natriumborhydrid.

US 3,131,132 A (Jefferson Chem. Comp., Inc.): Diskontinuierliche Umsetzung von überschüssigem DMA (1-2 eq) mit EO in Gegenwart von Wasser (3 - 15 eq) bei 50 - 100 °C; destillative Aufarbeitung (190 mbar) nach Einstellung des pH-Wertes auf 11,5 durch Säurezugabe.

JP 01160947 A (Mitsubishi Gas Chem. Co., Inc.): Wasserkatalysierte Synthese von DMEOA; Aufarbeitung durch destillative Hochsiederabtrennung (100 mbar); Hydrierung des Destillates an Ru/C; Reindestillation bei 100 mbar.

Nachteilig an diesen Verfahren sind die zusätzlichen Stoff- und Energiekosten durch die Nachbehandlung.

US 2004/0068143 offenbart ein Verfahren zur Herstellung von N,N-Diethylethanolamin durch kontinuierliche Umsetzung von Diethylamin und Ethylenoxid in Gegenwart von Wasser (siehe Beispiel 36). Die Temperatur der Umsetzung liegt im Bereich von 86-95°C (359-368°K) und die durchschnittliche Verweilzeit (VWZ) beträgt ungefähr 7 Min.. Die Sumpftemperatur liegt bei 102°C (375°K).

Spezielle Aufarbeitungs- bzw. Nachbehandlungsvarianten:

JP 61186349 A (Daicel Chem. Ind., Ltd.): Destillative Aufarbeitung (200 mbar) von Roh-DMEOA unter Zusatz von Harnstoff.

JP 57021351 A (Mitsubishi Gas Chem. Co., Inc.): Hydrierung von Roh-DMEOA an Ru/Al₂O₃.

EP 28 555 A1 (PCUK Produits Chim. Ugine Kuhlmann): Hydrierung von Roh-DMEOA an Raney-Nickel.

DE 30 40 732 A1 (PCUK Produits Chim. Ugine Kuhlmann): Reinigung von DMEOA durch Nachbehandlung mit Ammoniumsalzen.

US 6,774,264 B2 (Air Prod. and Chem., Inc.): Hydrierung von Roh-DMEOA Pd/Al₂O₃.

Nachteilig an diesen Verfahren sind auch hier die zusätzlichen Stoff- und Energiekosten durch die Nachbehandlung.

Wasserfreie Verfahren (T < 200 °C):

JP 01157938 A (Mitsubishi Gas Chem. Co., Inc.): Kontinuierliches Verfahren bei 150 °C/21 bar; Rückführung definierter Mengen des Reaktionsaustrages in den Reaktor (Autokatalyse).

DE 23 57 076 A1 (BASF AG): Kontinuierliches Verfahren; Rückführung definierter Mengen (0,01-0,5 fache Menge bzgl. EO plus DMA) des Reaktoraustrages (Autokatalyse). Farbstabile Produkte bei Umsetzungen < 160 °C.

Verfahren mit stabilisierenden Zusätzen:

US 3,567,779 A (Jefferson Chem. Comp., Inc.): Inhibierung der Verfärbung durch Zusatz von Mono- oder Diethanolamin zu DMEOA.

Nachteilig ist, dass stabilisierende Zusätze für viele Anwendungen von N,N-Dialkylethanolaminen, wie DMEOA, unerwünscht sind.

Wasserkatalysierte Verfahren ohne Nachbehandlung:

DD 203 534 A: Umsetzung von überschüssigem DMA (1,1-3,5 : 1) mit EO in Gegenwart katalytischer Mengen Wasser (0,02-0,15 eq) bei sehr milden Reaktionsbedingungen (50-90 °C); destillative Aufarbeitung mit max. Blasentemperatur von 90 °C.

Nachteilig ist, dass die Absenkung der Reaktionstemperatur unter 90 °C laut Literatur (Helv. Chim. Acta 52, 1969, Seite 408 - 418) zur Bildung quartärer Basen führt (→ Ausbeuteverluste). Quartäre Basen können in der anschließenden Aufarbeitung durch thermische Zersetzung Acetaldehyd freisetzen und zu schlechter Farbstabilität im Endprodukt führen.

EP 752 412 A1 (BASF AG) (Äquivalent DE 44 14 879 A): Umsetzung von überschüssigem DMA mit EO in Gegenwart von Wasser (2,5-50 Gew.-%, bevorzugt 8-25 Gew.-%) bei Reaktionstemperaturen ≥ 90 °C und destillative Aufarbeitung bei Temperaturen von max. 90 °C.

Nachteilig ist, dass die Sumpftemperatur bei der destillativen Aufarbeitung ≤ 90 °C gehalten werden muss.

Wasserkatalysierte Umsetzungen von Ethylenoxid mit Dimethylamin bei Reaktionstemperaturen > 90 °C sind Stand der Technik (z. B. EP 70 978 A; DE 44 14 879 A, s.o.). Die Farbstabilität von derart produziertem DMEOA muss jedoch entweder durch nachträgliche Behandlung des Rohaustrages (z. B. mit einem Reduktionsmittel/Nachhydrierung (US 6,774,264, s.o.) oder mit Säuren) oder durch Einhaltung maximaler Sumpftemperaturen (< 90 °C, DE 44 14 879 A) in der Reindestillation sichergestellt werden.

Der vorliegenden Erfindung lag die Aufgabe zugrunde, unter Überwindung von Nachteilen des Stands der Technik, ein verbessertes wirtschaftliches Verfahren zur Herstellung von N,N-Dialkyl-ethanolaminen, insb. DMEOA, aufzufinden. Das Verfahren sollte unter betriebssicheren Arbeitsbedingungen hochselektiv und mit hoher Raumzeitausbeute (RZA) die N,N-Dialkyl-ethanolamine mit hoher Farbstabilität liefern. Das Verfahren sollte dabei ohne Einsatz zusätzlicher Hilfsstoffe (stabilisierende Zusätze, die die Verfärbung inhibieren) auskommen.

[Raum-Zeit-Ausbeuten werden angegeben in ,Produktmenge / (Katalysatorvolumen ● Zeit)' (kg/(I_{Kat.} ● h)) und/oder ,Produktmenge / (Reaktorvolumen ● Zeit)' (kg/(I_{Reaktor} ● h)].

Demgemäß wurde ein Verfahren zur Herstellung eines N,N-Dialkyl-ethanolamins der Formel I mit hoher Farbstabilität 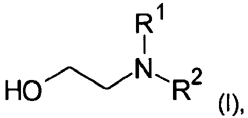 wobei R¹ und R² unabhängig voneinander eine C₁- bis C₈-Alkylgruppe bedeuten, durch Umsetzung von Ethylenoxid (EO) mit einem entsprechenden Dialkylamin (R¹R²NH) in Gegenwart von Wasser gefunden, welches dadurch gekennzeichnet ist, dass die Umsetzung in einem Reaktor kontinuierlich erfolgt, die Reaktionstemperatur im Bereich von 90 bis 180 °C und die Verweilzeit (VWZ) im Reaktor im Bereich von 1 bis 7 Min. liegt, der Reaktoraustrag bei einer Temperatur im Bereich von 80 bis 160 °C über eine Zeit im Bereich von 20 bis 1000 Min. thermisch behandelt und danach das N,N-Dialkyl-ethanolamin destillativ abgetrennt wird.

R¹ und R² bedeuten unabhängig voneinander eine lineare oder verzweigte C₁- bis C₈-Alkylgruppe, bevorzugt C₁- bis C₄-Alkylgruppe. Beispiele für solche Alkylgruppen sind Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, iso-Octyl, 2-Ethylhexyl. Besonders bevorzugt sind Methyl und Ethyl.

Besonders bevorzugt ist die Herstellung von N,N-Dimethyl-ethanolamin (DMEOA) durch Umsetzung von EO mit Dimethylamin (DMA).

Die Umsetzung erfolgt bevorzugt in Gegenwart von 2,5 bis 50 Gew.-%, besonders bevorzugt 5 bis 35 Gew.%, weiter bevorzugt 10 bis 30 Gew.-%, Wasser (bezogen auf das Reaktionsgemisch).

Dadurch werden im Gegensatz zu Verfahren unter Einsatz katalytischer Mengen Wasser (0,2 - 2,0 Gew.-% in DD 203 534 A; DE-PS 1 004 620 (Oxirane Ltd.), US 2,823,236 (A.J. Lowe et al.)) höhere Reaktionsgeschwindigkeiten sowie höhere Raum-Zeit-Ausbeuten erzielt.

Bevorzugt werden die Edukte in einem molaren Verhältnis von Dialkylamin : EO im Bereich von 1,1 bis 10, besonders bevorzugt 1,5 bis 9, weiter bevorzugt 3 bis 8, eingesetzt.

Dadurch wird die Bildung von quartären Basen und höher ethoxylierten Produkten weitgehend zurückgedrängt und dementsprechend eine hohe Selektivität erreicht.

Bevorzugt erfolgt die Umsetzung in einem flüssigkeitsgekühlten Rohrreaktor.

Besonders bevorzugt ist ein Doppelmantelreaktor. Der Reaktor kann im Gleich- oder Gegenstrom gekühlt werden. Bezüglich der Produktqualität (Farbzahl, Farbstabilität) hat sich die Gleichstromkühlung als vorteilhaft erwiesen. Daher fließt weiter bevorzugt die Kühlflüssigkeit im Gleichstrom durch den Doppelmantelreaktor.

Die Umsetzung erfolgt bevorzugt bei einem Absolutdruck im Bereich von 10 bis 40 bar, besonders bevorzugt 15 bis 30 bar.

Die Reaktionstemperatur liegt bevorzugt im Bereich von 110 bis 170 °C, besonders bevorzugt im Bereich von 125 bis 160 °C.

Die VWZ im Reaktor liegt bevorzugt im Bereich von 2 bis 5 Min., weiter bevorzugt im Bereich von 2 bis 4 Min..

Erfindungsgemäß wird der Reaktoraustrag bei einer Temperatur im Bereich von 80 bis 160 °C, bevorzugt 125 bis 155 °C, über eine Zeit im Bereich von 20 bis 1000 Min., bevorzugt 30 bis 700 Min., thermisch behandelt.

Bevorzugt wird vor der thermischen Behandlung des Reaktoraustrags unumgesetztes Dialkylamin destillativ abgetrennt.

Die thermische Behandlung kann in einem separaten Behälter (Verweilzeitbehälter) oder bevorzugt im Sumpfbehälter der Dialkylamin-Destillationskolonne erfolgen.

Nach der thermischen Behandlung erfolgt eine destillative Aufarbeitung zur Abtrennung des N,N-Dialkyl-ethanolamins.

Nach destillativer Abtrennung und Rückführung von überschüssigem Dialkylamin wird das Wasser abgetrennt und bevorzugt in das Verfahren zurückgeführt. Die Reindestillation des N,N-Dialkyl-ethanolamins ist diskontinuierlich möglich, wird aber bevorzugt kontinuierlich durchgeführt. In der Reindestillation werden Hochsieder (z. B. höher ethoxylierte Produkte und Vinyloxyethanol) als Bodenprodukt (Sumpfprodukt) ausgeschleust, das N,N-Dialkyl-ethanolamin, z. B. das DMEOA, wird entweder über Kopf oder bevorzugt über einen Seitenabzug abdestilliert. Leichtsieder wie Alkoxyethanol können in diesem Fall über Kopf abgetrennt werden. Besonders bevorzugt erfolgt also die destillative N,N-Dialkyl-ethanolamin - Abtrennung kontinuierlich in einer Seitenabzugskolonne, in der das N,N-Dialkyl-ethanolamin im Seitenabzug erhalten wird.

Erfindungsgemäß fällt das N,N-Dialkyl-ethanolamin der Formel I, z. B. DMEOA, in 99,00 - 99,99 %iger, besonders 99,50 bis 99,99 %iger, Reinheit an.

Im Besonderen liefert das erfindungsgemäße Verfahren das N,N-Dialkyl-ethanolamin der Formel I mit einer APHA-Farbzahl ≤ 15, besonders ≤ 13, weiter besonders ≤ 11, z. B. im Bereich von 1 bis 10.

Weiterhin liefert das erfindungsgemäße Verfahren im Besonderen das N,N-Dialkyl-ethanolamin der Formel I mit einer Farbstabilität derart, dass ein Erhitzen auf 60 °C für 24 h unter N₂ eine Erhöhung der APHA-Farbzahl um nur 0 bis 20 %, besonders 0 bis 15 %, bewirkt.

Die Ermittlung der APHA-Farbzahl erfolgt gemäß DIN-ISO 6271.

Die Spezifikation von im erfindungsgemäßen Verfahren eingesetztem EO lautet bevorzugt: > 99,9 % EO, < 60 ppm H₂O, < 50 ppm Acetaldehyd, < 20 ppm CO₂, < 20 ppm Essigsäure.

Die Spezifikation von im erfindungsgemäßen Verfahren bevorzugt eingesetztem DMA lautet im Besonderen: > 99,5 % DMA, < 0,1 % Monomethylamin (MMA), < 0,1 % NH₃, < 0,1 % MeOH, < 0,05 % (Trimethylamin) TMA, < 0,2 % H₂O.

ppm-Angaben beziehen sich auf die Masse (Massen-ppm).
Alle Druckangaben beziehen sich auf den Absolutdruck.

### Beispiele

### Beispiel 1

In einem 500 ml Rohrreaktor mit Druck- und Temperaturanzeige wurde ein auf 90 °C vorgewärmtes Gemisch aus Dimethylamin (3350 g/h; 74,5 mol/h) und Wasser (840 g/h; 46,7 mol/h; 21,3 Gew.-% bez. auf das Reaktionsgemisch) kontinuierlich mit 600 g/h (13,6 mol/h) Ethylenoxid umgesetzt. Infolge der Exothermie der Reaktion bildete sich im Reaktor ein Hot Spot. Durch externe Kühlung kühlte man den Reaktorinhalt auf 140 °C ab und hielt ihn bei dieser Temperatur, ggf. durch externe Wärmezufuhr, für 2,4-2,9 Min..

Nach destillativer Abtrennung und Rückführung von überschüssigem Dimethylamin (3 bar/Sumpftemperatur 140 °C / VWZ 35 Min.) und Wasser (Azeotrop mit Dimethylethanolamin; 400 mbar/Sumpftemperatur 110 °C) erhielt man 1275 g/h ethylenoxid-freies Rohprodukt mit folgender Zusammensetzung:

| | |
|---|---|
| Dimethylethanolamin: | 97,0 % |
| Dimethylaminodiglykol: | 1,2 % |
| Sonstige Produkte: | 1,8 % |

(%-Angaben beruhen auf GC-Analyse. Bedingungen: 30 m DB1 Säule mit folgendem Temperaturprogramm: 75 °C - 8 °C/Min. bis 280 °C - 12 Min. bei 280 °C.)
(VWZ = Verweilzeit)

Durch kontinuierliche destillative Aufarbeitung bei 50-60 mbar (Sumpftemperatur 70-100 °C) erhielt man Dimethylethanolamin in 99,8-99,9 %iger Reinheit mit einer Farbzahl von 5 APHA. Farbzahl nach 24 h / 60 °C: 5 APHA,

Farbzahl nach 3 Monaten bei Raumtemperatur unter Stickstoff: 10 APHA.

Leichtsiedende Nebenkomponente (Restwasser, Methoxyethanol, etc.) wurden dabei über Kopf, Hochsieder (Dimethylaminodiglykol, Vinyloxyethanol, etc.) über Sumpf und Dimethylethanolamin über einen Seitenabzug abgezogen.

Die Bestimmung der Farbstabilität erfolgt über folgenden Test:

Bestimmung der Farbzahl (APHA) nach Erhitzen des DMEAs für 24 h bei 60 °C unter Stickstoff.

### Beispiel 2

Die Reaktionsführung erfolgte wie in Beispiel 1 beschrieben. Vom Reaktoraustrag wurde zunächst überschüssiges Diethylamin abgetrennt. Im Vergleich zu Beispiel 1 lag die VWZ im Kolonnensumpf bei 14 Min. und die Sumpftemperatur bei 140 °C. Danach wurde der Kolonnensumpf in einen Behälter bei 90 °C gefahren. Die VWZ im Behälter betrug 660 Min.. Anschließend wurde Wasser abgetrennt und man erhielt 1275 g/h ethylenoxid-freies Rohprodukt mit einem > 97 %igen Gehalt an Dimethylethanolamin.

Durch kontinuierliche destillative Aufarbeitung bei 50-60 mbar (Sumpftemperatur 70-100 °C) erhielt man Dimethylethanolamin in 99,8-99,9 %iger Reinheit mit einer Farbzahl von 5 APHA.
Farbzahl nach 24 h / 60 °C: 5 APHA,
Farbzahl nach 3 Monaten bei Raumtemperatur unter Stickstoff: 10 APHA.
Leichtsiedende Nebenkomponente (Restwasser, Methoxyethanol, etc.) wurden dabei über Kopf, Hochsieder (Dimethylaminodiglykol, Vinyloxyethanol, etc.) über Sumpf und Dimethylethanolamin über einen Seitenabzug abgezogen.
(Bestimmung der Farbstabilität siehe Beispiel 1.)

### Vergleichsbeispiel:

Die Reaktionsführung erfolgte wie in Beispiel 1 beschrieben.

Vom Reaktoraustrag wurde zunächst überschüssiges Dimethylamin abgetrennt. Im Vergleich zu Beispiel 1 lag die VWZ im Kolonnensumpf bei 14 Min. und die Sumpftemperatur bei 140 °C. Anschließend wurde Wasser abgetrennt und man erhielt 1275 g/h ethylenoxid-freies Rohprodukt mit einem > 97 %igen Gehalt an Dimethylethanolamin.

Durch kontinuierliche Reindestillation bei 50-60 mbar (Sumpftemperatur 70-100 °C) erhielt man Dimethylethanolamin in 99,8-99,9 %iger Reinheit mit einer Farbzahl von 5 APHA.
Farbzahl nach 24 h / 60 °C: 50 APHA,
Farbzahl nach 3 Monaten bei Raumtemperatur unter Stickstoff: 55 APHA.

Das Wertprodukt Dimethylethanolamin wurde dabei wie in Beispiel 1 beschrieben über einen Seitenabzug abgetrennt. Leichtsiedende Nebenkomponente (Restwasser, Methoxyethanol, etc.) wurden über Kopf, Hochsieder (Dimethylaminodiglykol, Vinyloxyethanol, etc.) über Sumpf abgezogen.
(Bestimmung der Farbstabilität siehe Beispiel 1.)

Die Beispiele zeigen insb.:

Die Vorteile des Verfahrens gegenüber dem Stand der Technik liegen insb. einerseits in der hohen Reaktionsgeschwindigkeit, d.h. das Verfahren ist auch großtechnisch wirtschaftlich durchführbar. Die Bildung quartärer Ammoniumverbindungen und höher ethoxylierter Verbindungen ist unter den gefundenen Reaktionsbedingungen minimal, d.h. gegenüber Umsetzungen in Gegenwart katalytischer Mengen Wasser werden höhere Selektivitäten erreicht. Wird das Rohprodukt unter den gefundenen Bedingungen aufgearbeitet, so erhält man N,N-Dialkyl-ethanolamin, hier N,N-Dimethylethanolamin, mit hoher Farbstabilität, ohne Zusatz von Hilfsreagenzien (Hilfsstoffen), ohne Nachhydrierung und ohne Limitierung der Sumpftemperatur auf < 90 °C in der Reindestillation.

## Patentansprüche

1. Verfahren zur Herstellung eines N,N-Dialkyl-ethanolamins der Formel I mit hoher Farbstabilität 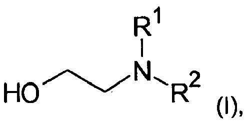 wobei R¹ und R² unabhängig voneinander eine C₁- bis C₈-Alkylgruppe bedeuten,
durch Umsetzung von Ethylenoxid (EO) mit einem entsprechenden Dialkylamin (R¹R²NH) in Gegenwart von Wasser, **dadurch gekennzeichnet, dass** die Umsetzung in einem Reaktor kontinuierlich erfolgt, die Reaktionstemperatur im Bereich von 90 bis 180 °C und die Verweilzeit (VWZ) im Reaktor im Bereich von 1 bis 7 Min. liegt, der Reaktoraustrag bei einer Temperatur im Bereich von 80 bis 160 °C über eine Zeit im Bereich von 20 bis 1000 Min. thermisch behandelt und danach das N,N-Dialkyl-ethanolamin destillativ abgetrennt wird.

2. Verfahren nach Anspruch 1, **dadurch gekennzeichnet, dass** vor der thermischen Behandlung des Reaktoraustrags unumgesetztes Dialkylamin destillativ abgetrennt wird.

3. Verfahren nach Anspruch 2, **dadurch gekennzeichnet, dass** die thermische Behandlung im Sumpfbehälter der Dialkylamin-Destillationskolonne erfolgt.

4. Verfahren nach einem der vorhergehenden Ansprüche, **dadurch gekennzeichnet, dass** die Reaktionstemperatur im Bereich von 110 bis 170 °C liegt.

5. Verfahren nach einem der vorhergehenden Ansprüche, **dadurch gekennzeichnet, dass** die VWZ im Reaktor im Bereich von 2 bis 5 Min. liegt.

6. Verfahren nach einem der vorhergehenden Ansprüche, **dadurch gekennzeichnet, dass** der Reaktoraustrag bei einer Temperatur im Bereich von 125 bis 155 °C thermisch behandelt wird.

7. Verfahren nach einem der vorhergehenden Ansprüche, **dadurch gekennzeichnet, dass** der Reaktoraustrag über eine Zeit im Bereich von 30 bis 700 Min. thermisch behandelt wird.

8. Verfahren nach einem der vorhergehenden Ansprüche, **dadurch gekennzeichnet, dass** die Umsetzung in einem flüssigkeitsgekühlten Rohrreaktor erfolgt.

9. Verfahren nach dem vorhergehenden Anspruch, **dadurch gekennzeichnet, dass** die Umsetzung in einem Doppelmantelreaktor erfolgt.

10. Verfahren nach dem vorhergehenden Anspruch, **dadurch gekennzeichnet, dass** die Kühlflüssigkeit im Gleichstrom durch den Doppelmantelreaktor fließt.

11. Verfahren nach einem der vorhergehenden Ansprüche, **dadurch gekennzeichnet, dass** die Umsetzung in Gegenwart von 2,5 bis 50 Gew.-% Wasser (bezogen auf das Reaktionsgemisch) erfolgt.

12. Verfahren nach einem der vorhergehenden Ansprüche, **dadurch gekennzeichnet, dass** die Edukte in einem molaren Verhältnis von Dialkylamin : EO im Bereich von 1,1 bis 10 eingesetzt werden.

13. Verfahren nach einem der vorhergehenden Ansprüche, **dadurch gekennzeichnet, dass** die Umsetzung bei einem Absolutdruck im Bereich von 10 bis 40 bar erfolgt.

14. Verfahren nach einem der vorhergehenden Ansprüche, **dadurch gekennzeichnet, dass** die destillative N,N-Dialkyl-ethanolamin - Abtrennung kontinuierlich in einer Seitenabzugskolonne erfolgt, in der das N,N-Dialkyl-ethanolamin im Seitenabzug erhalten wird.

15. Verfahren nach einem der vorhergehenden Ansprüche zur Herstellung eines N,N-Dialkyl-ethanolamins der Formel I mit einer APHA-Farbzahl ≤ 15.

16. Verfahren nach einem der vorhergehenden Ansprüche zur Herstellung eines N,N-Dialkyl-ethanolamins der Formel I mit einer Farbstabilität derart, dass ein Erhitzen auf 60 °C für 24 h unter N₂ eine Erhöhung der APHA-Farbzahl um nur 0 bis 20 % bewirkt.

17. Verfahren nach einem der vorhergehenden Ansprüche zur Herstellung eines N,N-Dialkyl-ethanolamins der Formel I, wobei R¹ und R² unabhängig voneinander eine C₁- bis C₄-Alkylgruppe bedeuten.

18. Verfahren nach einem der vorhergehenden Ansprüche zur Herstellung von N,N-Dimethylethanolamin (DMEOA) durch Umsetzung von EO mit Dimethylamin (DMA).

## Claims

1. A process for preparing an N,N-dialkylethanolamine of the formula I having high color stability 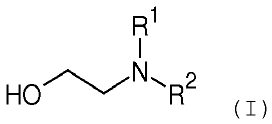 where R¹ and R² are each independently a C₁- to C₈-alkyl group, by reacting ethylene oxide (EO) with a corresponding dialkylamine (R¹R²NH) in the presence of water, wherein the reaction is effected continuously in a reactor, the reaction temperature is in the range from 90 to 180°C and the residence time (RT) in the reactor is in the range from 1 to 7 min, the reactor output is treated thermally at a temperature in the range from 80 to 160°C over a period in the range from 20 to 1000 min, and then the N,N-dialkylethanolamine is removed by distillation.

2. The process according to claim 1, wherein the thermal treatment of the reactor output is preceded by distillative removal of unconverted dialkylamine.

3. The process according to claim 2, wherein the thermal treatment is effected in the bottoms vessel of the dialkylamine distillation column.

4. The process according to any of the preceding claims, wherein the reaction temperature is in the range from 110 to 170°C.

5. The process according to any of the preceding claims, wherein the RT in the reactor is in the range from 2 to 5 min.

6. The process according to any of the preceding claims, wherein the reactor output is treated thermally at a temperature in the range from 125 to 155°C.

7. The process according to any of the preceding claims, wherein the reactor output is treated thermally over a period in the range from 30 to 700 min.

8. The process according to any of the preceding claims, wherein the reaction is effected in a liquid-cooled tubular reactor.

9. The process according to the preceding claim, wherein the reaction is effected in a jacketed reactor.

10. The process according to the preceding claim, wherein the cooling liquid flows through the jacketed reactor in cocurrent.

11. The process according to any of the preceding claims, wherein the reaction is effected in the presence of 2.5 to 50% by weight of water (based on the reaction mixture).

12. The process according to any of the preceding claims, wherein the reactants are used in a molar ratio of dialkylamine:EO in the range from 1.1 to 10.

13. The process according to any of the preceding claims, wherein the reaction is effected at an absolute pressure in the range from 10 to 40 bar.

14. The process according to any of the preceding claims, wherein the distillative N,N-dialkyl-ethanolamine removal is effected continuously in a side draw column in which the N,N-dialkylethanolamine is obtained in the side draw.

15. The process according to any of the preceding claims for preparing an N,N-dialkylethanolamine of the formula I having an APHA color number of ≤ 15.

16. The process according to any of the preceding claims for preparing an N,N-dialkylethanolamine of the formula I having such a color stability that heating to 60°C for 24 h under N₂ causes an increase in the APHA color number by only 0 to 20%.

17. The process according to any of the preceding claims for preparing an N,N-dialkylethanolamine of the formula I, where R¹ and R² are each independently a C₁-to C₄-alkyl group.

18. The process according to any of the preceding claims for preparing N,N-dimethylethanolamine (DMEOA) by reacting EO with dimethylamine (DMA).

## Revendications

1. Procédé de fabrication d'une N,N-dialkyl-éthanolamine de formule I présentant une grande stabilité de la couleur 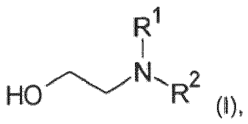 dans laquelle R¹ et R² signifient indépendamment l'un de l'autre un groupe alkyle en C₁ à C₈,
par mise en réaction d'oxyde d'éthylène (EO) avec une dialkylamine correspondante (R¹R²NH) en présence d'eau, **caractérisé en ce que** la réaction a lieu dans un réacteur en continu, la température de réaction se situe dans la plage allant de 90 à 180 °C et le temps de séjour (VWZ) dans le réacteur se situe dans la plage allant de 1 à 7 minutes, la sortie du réacteur est traitée thermiquement à une température dans la plage allant de 80 à 160 °C pendant une durée dans la plage allant de 20 à 1 000 minutes, puis la N,N-dialkyl-éthanolamine est séparée par distillation.

2. Procédé selon la revendication 1, **caractérisé en ce que** la dialkylamine non réagie est séparée par distillation avant le traitement thermique de la sortie du réacteur.

3. Procédé selon la revendication 2, **caractérisé en ce que** le traitement thermique a lieu dans le contenant de fond de la colonne de distillation de la dialkylamine.

4. Procédé selon l'une quelconque des revendications précédentes, **caractérisé en ce que** la température de réaction se situe dans la plage allant de 110 à 170 °C.

5. Procédé selon l'une quelconque des revendications précédentes, **caractérisé en ce que** le VWZ dans le réacteur se situe dans la plage allant de 2 à 5 minutes.

6. Procédé selon l'une quelconque des revendications précédentes, **caractérisé en ce que** la sortie du réacteur est traitée thermiquement à une température dans la plage allant de 125 à 155 °C.

7. Procédé selon l'une quelconque des revendications précédentes, **caractérisé en ce que** la sortie du réacteur est traitée thermiquement pendant une durée dans la plage allant de 30 à 700 minutes.

8. Procédé selon l'une quelconque des revendications précédentes, **caractérisé en ce que** la réaction a lieu dans un réacteur tubulaire refroidi par un liquide.

9. Procédé selon la revendication précédente, **caractérisé en ce que** la réaction a lieu dans un réacteur à double enveloppe.

10. Procédé selon la revendication précédente, **caractérisé en ce que** le liquide de refroidissement s'écoule à co-courant dans le réacteur à double enveloppe.

11. Procédé selon l'une quelconque des revendications précédentes, **caractérisé en ce que** la réaction a lieu en présence de 2,5 à 50 % en poids d'eau (par rapport au mélange réactionnel).

12. Procédé selon l'une quelconque des revendications précédentes, **caractérisé en ce que** les réactifs sont utilisés en un rapport molaire dialkylamine:EO de 1,1 à 10.

13. Procédé selon l'une quelconque des revendications précédentes, **caractérisé en ce que** la réaction a lieu à une pression absolue dans la plage allant de 10 à 40 bar.

14. Procédé selon l'une quelconque des revendications précédentes, **caractérisé en ce que** la séparation par distillation de la N,N-dialkyl-éthanolamine a lieu en continu dans une colonne à soutirage latéral dans laquelle la N,N-dialkyl-éthanolamine est obtenue par le soutirage latéral.

15. Procédé selon l'une quelconque des revendications précédentes, pour la fabrication d'une N,N-dialkyl-éthanolamine de formule I ayant un indice de couleur APHA ≤ 15.

16. Procédé selon l'une quelconque des revendications précédentes, pour la fabrication d'une N,N-dialkyl-éthanolamine de formule I présentant une stabilité de la couleur telle qu'un chauffage à 60 °C pendant 24 h sous N₂ ne provoque qu'une élévation de l'indice de couleur APHA de 0 à 20 %.

17. Procédé selon l'une quelconque des revendications précédentes, pour la fabrication d'une N,N-dialkyl-éthanolamine de formule I, dans laquelle R¹ et R² signifient indépendamment l'un de l'autre un groupe alkyle en C₁ à C₄.

18. Procédé selon l'une quelconque des revendications précédentes, pour la fabrication de N,N-diméthyl-éthanolamine (DMEOA) par mise en réaction d'EO avec de la diméthylamine (DMA).
